(19) Europäisches Patentamt
European Patent Office
Office européen des brevets

(11) EP 3 376 159 A1

(12) EUROPEAN PATENT APPLICATION

(43) Date of publication:
19.09.2018  Bulletin 2018/38

(51) Int Cl.:
G01B 21/32 (2006.01)  G01B 7/16 (2006.01)
A61J 13/00 (2006.01)  G01B 13/24 (2006.01)
A61B 5/03 (2006.01)

(21) Application number: 17161232.8

(22) Date of filing: 16.03.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Nokia Technologies Oy
02610 Espoo (FI)

(72) Inventors:
• Rajala, Satu
  02680 Espoo (FI)
• Blomqvist, Kim
  02650 Espoo (FI)
• Huttunen, Janne
  02610 Espoo (FI)
• Müller, Kiti
  00430 Helsinki (FI)
• Kärkkäinen, Leo
  00100 Helsinki (FI)

(74) Representative: Espatent Oy
Kaivokatu 10 D
00100 Helsinki (FI)

(54) **APPARATUS FOR MONITORING A SUBJECT, SUCH AS A BABY**

(57)  An apparatus including a body made of force-conveying material, wherein the body comprises a nozzle part and a base part, the nozzle part protruding from the base part, and wherein the nozzle part is configured to fit at least partly into a mouth of a subject for feeding the subject, and at least one deformation sensor located in the base part, the deformation sensor being configured to sense, at the base part, deformation conveyed from the nozzle part through the force-conveying material.

Fig. 3

## Description

### TECHNICAL FIELD

The present application generally relates to arrangements for monitoring a subject such as a baby and to monitoring sucking force or sucking behaviour of the subject.

### BACKGROUND

This section illustrates useful background information without admission of any technique described herein representative of the state of the art.

There is a need for devices to assess oral feeding ability of babies. Sucking skills of a baby can provide valuable insights into the baby's health and its future development.

### SUMMARY

Various aspects of examples of the invention are set out in the claims.

According to a first example aspect of the present invention, there is provided an apparatus comprising:

a body made of force-conveying material, wherein the body comprises a nozzle part and a base part, the nozzle part protruding from the base part, and wherein the nozzle part is configured to fit at least partly into a mouth of a subject for feeding the subject, and at least one deformation sensor located in the base part, the deformation sensor being configured to sense, at the base part, deformation conveyed from the nozzle part through the force-conveying material. to detect deformation of the nozzle part.

In an embodiment, the deformation sensor produces a measurement output indicative of said sensed deformation. In an embodiment, the measurement output is usable for analyzing forces applied to the nozzle part. In an embodiment, the measurement output is usable for determining deformation of the nozzle part.

In an embodiment, said nozzle part and said base part are seamlessly attached to each other.

In an embodiment, said nozzle part forms a cavity and said cavity is free from said deformation sensors.

In an embodiment, said nozzle part is free from said deformation sensors.

In an embodiment the base part is configured to be in contact with breast skin surrounding a nipple of a user. In another embodiment, the base part is configured to extend in a plane generally parallel with the skin of the user, either with or without direct contact with the skin. According to yet another embodiment, the base part is configured to be in contact with an areola of the user or with an areola and the surrounding skin of the user.

In an embodiment, said deformation sensor is a strain gauge.

In an embodiment, said strain gauge is one of: a nanoparticle based strain gauge, an elastomer strain gauge, a piezoelectric polymer strain gauge, a semiconductor strain gauge, or a fiber-optic strain gauge.

In an embodiment, said force-conveying material is flexible silicone.

In an embodiment, said force-conveying material comprises one or more fluid cavities that extend from the nozzle part to the base part, the apparatus configured such that the fluid in the cavities conveys to the base part a force applied to the nozzle part.

In an embodiment, the apparatus is a nipple shield. In another embodiment, the apparatus is a nipple of a bottle.

In an embodiment, the apparatus is a nipple shield and the nozzle part forms a nipple cavity configured to receive a nipple of a user and the base part is configured to be in contact with breast skin surrounding the nipple.

In an embodiment, the apparatus comprises a plurality of deformation sensors located in the base part.

In an embodiment, the deformation sensor is configured to measure deformation at a plurality of locations in the base part and to produce a plurality of measurement outputs.

In an embodiment, the deformation sensor is embedded in the force-conveying material.

In an embodiment, the deformation sensor is attached to a surface of the base part.

In an embodiment, the base part comprises a pocket configured to receive the deformation sensor.

According to a second example aspect of the present invention, there is provided a method comprising:

sensing deformation conveyed from a nozzle part of an apparatus comprising a body made of force-conveying material, wherein the body comprises the nozzle part and a base part, the nozzle part protruding from the base part, and wherein the nozzle part is configured to fit at least partly into a mouth of a subject for feeding the subject, and performing the deformation sensing by at least one deformation sensor located in the base part, the deformation sensor being configured to sense, at the base part, deformation conveyed from the nozzle part through the force-conveying material.

According to a third example aspect of the present invention, there is provided an apparatus comprising:

a body made of force-conveying material, wherein the body comprises a nozzle part and a base part, the nozzle part protruding from the base part,

and wherein the nozzle part is configured to fit at least partly into a mouth of a subject for feeding the subject, wherein

the base part comprises a pocket configured to receive a deformation sensor, the deformation sensor being configured to sense, at the base part, deformation conveyed from the nozzle part through the force-conveying material.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

## BRIEF DESCRIPTION OF THE DRAWINGS

For a more complete understanding of example embodiments of the present invention, reference is now made to the following descriptions taken in connection with the accompanying drawings in which:

Figs. 1A and 1B are simplified illustrations of nipple shields;

Fig. 2 is a simplified illustration of a nipple of a baby bottle;

Figs. 3-8 are simplified illustrations of example apparatuses; and

Fig. 9 shows a flow chart of a process of an example embodiment.

## DETAILED DESCRIPTON OF THE DRAWINGS

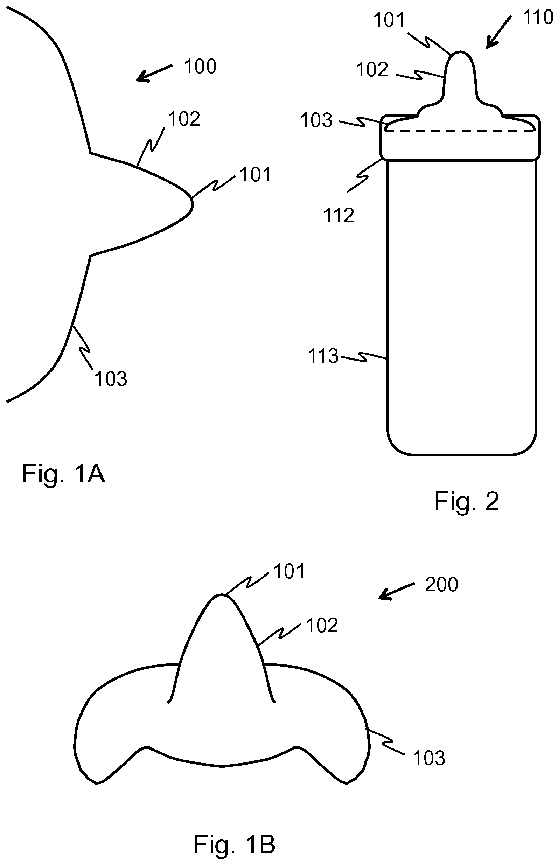

Example embodiments of the present invention and its potential advantages are understood by referring to Figs. 1 through 9 of the drawings. In this document, like reference signs denote like parts or steps.

In various example embodiments of the invention there is provided a new device suited for non-invasive monitoring of babies and particularly for monitoring sucking behaviour of babies. Sucking behaviour of babies can be analysed based on determined sucking forces of babies.

In the following, various example embodiments are discussed in connection with nipple shields. A nipple shield is a small device that is used for covering a nipple of a mother during a breastfeeding event. A nipple shield is made of flexible silicone, for example. In addition to nipple shields, the embodiments of the invention can be applied for example to nipples of baby bottles or other containers.

Figs. 1A and 1B are simplified illustrations of nipple shields 100 and 200. Fig. 2 is a simplified illustration of a nipple 110 of a baby bottle. Embodiments of the invention may be implemented in the nipple shields 100 and 200 of Figs. 1A and 1B or in the nipple 110 of Fig. 2. The nipple shields 100 and 200 and the nipple 110 are referred to with a term nipple element in the following. The nipple element comprises a base part 103 and a nozzle part 102. The nozzle part 102 protrudes from the base part 103. In an embodiment the nozzle part 102 forms a cavity that extends from the base part. In an embodiment, the base part 103 forms a brim that surrounds the nozzle part 102. The base part 103 and the nozzle part 102 may be seamlessly attached to each other. In an embodiment the seamless attachment between the base part and the nozzle part refers to the nipple element being made of a single piece of material for example by injection molding. The base part 103 of the nipple shield 200 of Fig. 1B has a cut-out section. Some nipple shields are designed to include a cut-out section for example for ergonomic reasons, but a nipple shield may also be provided without a cut-out section, and the existence of such cut-out section is not necessary for implementation of embodiments of the invention.

In the nipple shield embodiments, the nozzle part 102 forms a nipple cavity configured to receive a nipple of a user (mother). In an embodiment the base part is configured to be in contact with breast skin surrounding the nipple of the user. In another embodiment, the base part is configured to extend in a plane generally parallel with the skin of the user (mother), either with or without direct contact with the skin. According to yet another embodiment, the base part is configured to be in contact with an areola of the user (mother) or with an areola and the surrounding skin of the user (mother).

The nipple elements 100, 200 and 110 are suited for feeding a subject, e.g. a baby. The nozzle part 102 is suited for being put into a mouth of the baby and the baby is fed through the tip 101 of the nozzle part. There are one or more apertures (not shown in the Fig) in the tip area 101 of the nozzle part to allow fluid (e.g. milk) to flow through the nipple element 100, 200 and 110.

Fig. 2 shows the nipple element 110 attached to a baby bottle. The bottle comprises a fluid container 113 and a collar part 112. The base part 103 of the nipple element 110 is connected to the fluid container 113 so that fluid (e.g. milk) in the fluid container 113 is allowed to flow to the nipple element 110 and through the apertures in the tip area 101 of the nipple element 110 to a mouth of a subject that is being fed. In the shown example, the collar part 112 is configured to attach the nipple element 110 to the fluid container 113. Also other configurations for attaching the nipple element 110 to a fluid container may be used. In an embodiment, the base part 103 of the nipple element is configured to provide mechanical connection between the nozzle part 102 and a fluid container.

In embodiments of the invention there is at least one deformation sensor located in the base part of the nipple element. As the nipple element and particularly the nozzle part thereof is deformed due to a baby or other subject sucking the nozzle part the deformation is sensed by the deformation sensor. The nipple shield is made of suitable force-conveying material, such as thin flexible silicone, for example. Force-conveying material refers to a material in which a force applied to the material at one location is conveyed, at least in part, in the material so that the force can be sensed at other locations, too. That is, the force-conveying material transmits the forces applied to the nozzle part also to the base part. Therefore deformation of the nozzle part causes some deformation also in the base part. Thereby the deformation sensor in the base part can be used for determining deformation of the nozzle part and for analyzing forces applied to the nozzle part.

The deformation sensor in embodiments of the invention is for example a strain gauge. Examples of suitable strain gauge types include: a nanoparticle based strain gauge, an elastomer strain gauge, a piezoelectric polymer strain gauge, a semiconductor strain gauge, and a fiber-optic strain gauge. In an embodiment, the deformation sensor is made of a heat-resistant material, e.g. material that endures boiling water.

Herein it is noted that deformation refers to a change of dimensions expressed in absolute terms, whereas strain is a relative measure of the same.

The deformation sensor may be attached to the inner or outer surface of the base part for example with a suitable adhesive or the deformation sensor may be integrated in the base part or there may be a pocket suited for receiving the deformation sensor. In an embodiment there may be a moisture protection layer that covers the deformation sensor. This is suited for example to embodiments where the deformation sensor is attached to the inner surface of the nipple element. Also other attachment types may be used. In an example embodiment, when the nozzle part is deformed, the deformation is transmitted to the base part and the strain gauge is deformed causing electrical resistance of the strain gauge to change. A deformation sensor produces a measurement output indicative of deformation. The measurement output produced by the deformation sensor (e.g. strain gauge) can be related to or converted to a deformation using a sensitivity coefficient. The sensitivity coefficient defines a relationship between the measured quantity (given as the measurement output of the deformation sensor) and the deformation. Depending on the type of the deformation sensor the measured quantity may be for example electrical resistance or electrical charge. The deformation may be further converted into an absolute force with a calibration coefficient determined in a calibration procedure, for example when absolute forces acting on the nozzle part need to be determined.

In an example embodiment deformation is measured using a strain gauge in which electrical resistance depends on deformation. The sensitivity coefficient of a strain gauge is referred to as its gauge factor (GF). A gauge factor defines a relationship between measured electrical resistance and deformation. The gauge factor is defined as

$$GF = (\Delta R/R_0)/\varepsilon,$$

where

$R_0$ = unstrained resistance of strain gauge,
$\Delta R$ = change in strain gauge resistance,
$\varepsilon$ = strain = $\Delta L/L_0$, where $L_0$ = original length and $\Delta L$ = change in length.

In an embodiment strain gauges with a high GF value are used. The GF value may be for example 100 or higher. In some embodiments the GF value may be 100-2000. It may be estimated that the sucking force of a baby is around 1-2 N. With a GF value of 1000 and estimating that a sucking force of around 0.1 N is transmitted to a strain gauge located in the base part of the nipple element, the change in strain gauge resistance would be around 10 $\Omega$ (estimated $R_0$ = 100 $\Omega$, E = 2*10^9 N/m2, where E is Young's modulus, and A=width*thickness=5mm*100$\mu$m, where A is the surface area of the strain gauge). This example demonstrates how a measurement output representing sucking force is detected in a case, where sucking force applied to the nozzle part is partially conveyed to the base part.

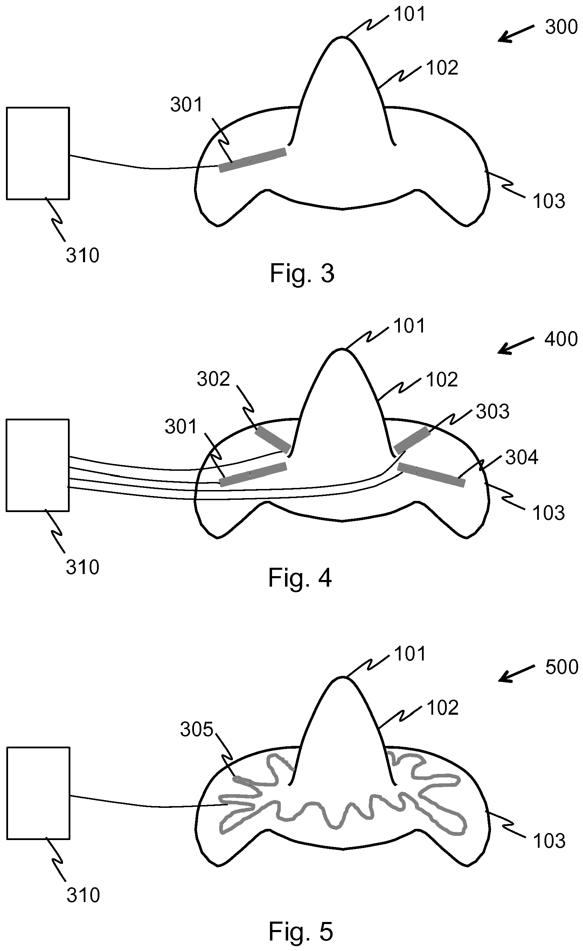

Figs. 3-8 are simplified illustrations of example apparatuses 300-800. The apparatuses 300-800 are nipple shields that comprise a base part or a brim 103, a nozzle part 102 and a tip 101 similarly to Figs. 1A, 1B and 2. The nipple shield 300-800 is made of suitable force-conveying material. The example embodiments discussed in connection with Figs. 3-8 may be applied to other nipple elements in addition to a nipple shield, too.

The nipple shield 300 of Fig. 3 comprises a deformation sensor 301 located in the base part 103 of the nipple shield. The deformation sensor 301 is located outside the nozzle part 102. That is, the nozzle part 102 is free from deformation sensors in this embodiment. In an embodiment, the nozzle part 102 forms a cavity and the cavity is free from deformation sensors. The deformation sensor 301 produces a measurement output indicative of deformations sensed by the deformation sensor 301. The deformation sensor may be attached to the surface of the base part 103 or integrated into the material forming the nipple shield 300. Additionally, Fig. 3 shows a processing unit 310 that is coupled to the deformation sensor 301. The processing unit 310 may be an electronics module comprising for example a housing, power source, processor, memory and other electronic components. The processing unit 310 may also me a mobile electronic device such as a smartphone, or a laptop or desktop computer. The processing unit 310 may be connected to the deformation sensor 301 with a wired connection or a wireless connection. The processing unit 310 may comprise functionality to analyse the deformations sensed by the deformation sensor 301 and/or functionality to store the measurement results for further processing. Analysis of the deformations sensed by the deformation sensor 301 may be used to support the detection of health and welfare concerns using various methods as described for example in a research paper Tamilia, E. et al (2014). Technological Solutions and Main Indices for the Assessment of Newborns' Nutritive Sucking: a Review. Sensors, 14(1), 634-658.

The nipple shield 400 of Fig. 4 comprises multiple deformation sensors 301-304 located in the base part 103 of the nipple shield on different sides of the nozzle part so that the deformation sensors 301-304 surround the nozzle part 102. By having the deformation sensors on different sides of the device, one is able to obtain more detailed information about deformations of the nozzle part and thereby more information about a sucking profile of the baby that is being fed. The deformation sensors 301-304 are located outside the nozzle part 102. That is, the nozzle part 102 is free from deformation sensors in this embodiment. In an embodiment, the nozzle part 102 forms a cavity and the cavity is free from deformation sensors.

Additionally, Fig. 4 shows a processing unit 310 that is coupled to the deformation sensors 301-304. The processing unit 310 may comprise functionality to analyse the deformations sensed by the deformation sensors 301-304 and/or functionality to store the measurement results for further processing.

The nipple shield 500 of Fig. 5 comprises a deformation sensor 305 located in the base part 103 of the nipple shield. The deformation sensor 305 circles around the nozzle part 102. The deformation sensor 305 is located outside the nozzle part 102. That is, the nozzle part 102 is free from deformation sensors in this embodiment. In an embodiment, the nozzle part 102 forms a cavity and the cavity is free from deformation sensors. Similarly to the arrangement of Fig. 4 also the deformation sensor 305 allows deformation sensing on different sides of the device. In this way one is able to obtain more detailed information about deformations of the nozzle part and thereby more information about a sucking profile of the baby that is being fed. The deformation sensor 305 may comprise a plurality of electrodes at different locations along its length to enable deformation to be measured at the respective locations.

Additionally, Fig. 5 shows a processing unit 310 that is coupled to the deformation sensor 305. The processing unit 310 may comprise functionality to analyse the deformations sensed by the deformation sensor 305 and/or functionality to store the measurement results for further processing. Fig. 5 shows one output from the deformation sensor, but alternatively, there may be more than one output from the deformation sensor 305 to obtain measurement results from different electrode locations. Equally the deformation sensors 301-304 of Figs. 3 and 4 may provide more than one output.

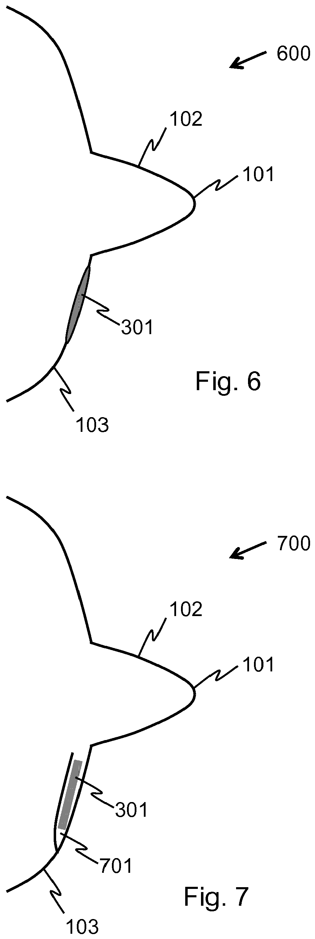

The nipple shield 600 of Fig. 6 comprises a deformation sensor 301 located in the base part 103 of the nipple shield. The deformation sensor 301 is located outside the nozzle part 102. That is, the nozzle part 102 is free from deformation sensors in this embodiment. In an embodiment, the nozzle part 102 forms a cavity and the cavity is free from deformation sensors. In the shown example, the deformation sensor 301 is integrated or moulded in the material of the base part 103.

The nipple shield 700 of Fig. 7 comprises a deformation sensor 301 located in the base part 103 of the nipple shield. The deformation sensor 701 is located outside the nozzle part 102. That is, the nozzle part 102 is free from deformation sensors in this embodiment. In an embodiment, the nozzle part 102 forms a cavity and the cavity is free from deformation sensors. In the shown example, the base part 103 comprises a pocket 701 formed on an inner surface of the base part and the deformation sensor 301 is located in the pocket 701. The pocket 701 may also be formed on an outer surface of the base part 301. Such arrangement allows detaching the deformation sensor 301 from the nipple shield. Thereby the deformation sensor 301 may be used with different nipple shields.

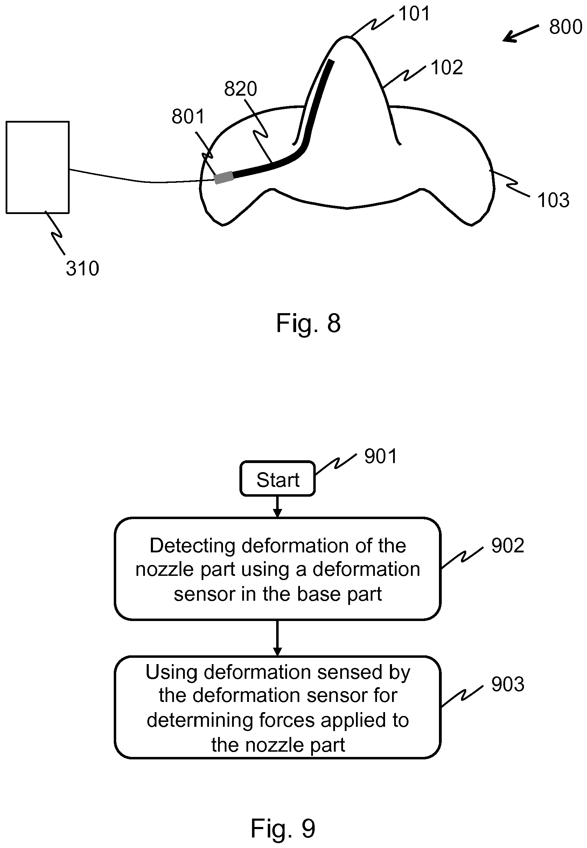

The nipple shield 800 of Fig. 8 comprises a longitudinal fluid cavity 820 that extends from the nozzle part 102 to the base part 103. For the sake of simplicity, only one fluid cavity 820 is shown, but there may be any suitable number of fluid cavities. Additionally there is a deformation sensor 801 located in the base part 103. The deformation sensor 801 is located outside the nozzle part 102. That is, the nozzle part 102 is free from deformation sensors in this embodiment. In an embodiment, the nozzle part 102 forms a cavity and the cavity is free from deformation sensors. In this example, the deformation sensor 801 may be a pressure sensor that senses pressure changes in the fluid of the fluid cavity 820. As the nozzle part 102 is deformed by sucking forces during a feeding event, the deformation causes pressure changes in the fluid cavity 820 and these pressure changes are used for detecting the deformation.

Additionally, Fig. 8 shows a processing unit 310 that is coupled to the deformation sensor 801. The processing unit 310 may comprise functionality to analyse the deformations sensed by the deformation sensor 801 and/or functionality to store the measurement results for further processing.

In an embodiment, the processing unit 310 of Figs. 3-5 and 8 is detachable from the deformation sensor(s). In this way the processing unit 310 can be used with multiple nipple shields. By having electronic components that do not endure heat in a detachable processing unit allows sterilizing the nipple shield e.g. by cleaning it in boiling water.

In an embodiment, the processing unit 310 of Figs. 3-5 and 8 may be configured to send the measurement data to a separate unit for further processing. I.e. the processing unit 310 does not necessarily process the data except only forwards it to a separate unit. The measurement data may be sent over a wireless connection, for example. In an embodiment a loop type deformation sensor 305 of Fig. 5 may be used as an antenna to send the measurement data wirelessly.

It is to be noted that sizes of deformation sensors and locations of deformation sensors in Figs. 3-8 are only illustrative and do not necessarily reflect actual sizes or locations. Additionally, it is noted that details disclosed in connection with one figure or one embodiment may be combined to embodiments of other figures.

Fig. 9 shows a flow chart of a process of an example embodiment. The process may be implemented for example in one of the apparatuses of Figs. 3-8 or the like. The process concerns using a device suited for feeding a subject (e.g. a baby). The device comprises a body made of force-conveying material, and the body comprises a nozzle part and a base part. The nozzle part protrudes from the base part and the nozzle part is configured to fit into a mouth of the subject for feeding the subject. The process comprises the following phases:

901: The process is started.

902: Deformation of the nozzle part or deformation conveyed from the nozzle part is sensed using a deformation sensor located in the base part.

903: Sensed deformation is used for determining forces applied to the nozzle part. The determined forces may then be used for analysing sucking behaviour of the subject. This determining and/or analysing may take place for example in the processing unit 310 of Figs. 3-5 and 8 or elsewhere in a suitable data processing environment.

Another technical effect of one or more of the example embodiments disclosed herein is a device structure that allows easy cleaning of the device, e.g. sterilisation by boiling, to maintain hygiene.

Another technical effect of one or more of the example embodiments disclosed herein is that fluid flow through the device is undisturbed as the cavity forming the nozzle part is free from deformation sensors.

Another technical effect of one or more of the example embodiments disclosed herein is that flexibility of the nozzle part is not affected by presence of deformation sensors as the nozzle part is free from deformation sensors.

Another technical effect of one or more of the example embodiments disclosed herein is improved safety. As the nozzle part is free from deformation sensors no electronic components are put into the mouth of a baby. It is also less likely that the baby would accidentally break the deformation sensor with his/her mouth (teeth or gums).

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications which may be made without departing from the scope of the present invention as defined in the appended claims.

**Claims**

1. An apparatus comprising:

   a body made of force-conveying material, wherein the body comprises a nozzle part and a base part, the nozzle part protruding from the base part, and wherein the nozzle part is configured to fit at least partly into a mouth of a subject for feeding the subject, and
   at least one deformation sensor located in the base part, the deformation sensor being configured to sense, at the base part, deformation conveyed from the nozzle part through the force-conveying material.

2. The apparatus of claim 1, wherein said nozzle part and said base part are seamlessly attached to each other.

3. The apparatus of claim 1 or 2, wherein said nozzle part forms a cavity and said cavity is free from said deformation sensors.

4. The apparatus of claim 1 or 2, wherein said nozzle part is free from said deformation sensors.

5. The apparatus of any preceding claim, wherein said deformation sensor is a strain gauge.

6. The apparatus of claim 5, wherein said strain gauge is one of: a nanoparticle based strain gauge, an elastomer strain gauge, a piezoelectric polymer strain gauge, a semiconductor strain gauge, or a fiber-optic strain gauge.

7. The apparatus of any preceding claim, wherein said force-conveying material is flexible silicone.

8. The apparatus of any preceding claim, wherein said force-conveying material comprises one or more fluid cavities that extend from the nozzle part to the base part, the apparatus configured such that the fluid in the cavities conveys to the base part a force applied to the nozzle part.

9. The apparatus of any preceding claim, wherein the apparatus is a nipple shield or a nipple of a bottle.

10. The apparatus of any preceding claim, wherein the apparatus is a nipple shield and the nozzle part forms a nipple cavity configured to receive a nipple of a user and the base part is configured to be in contact with breast skin surrounding the nipple.

11. The apparatus of any preceding claim, wherein the apparatus comprises a plurality of deformation sensors located in the base part.

12. The apparatus of any preceding claim, wherein the deformation sensor is configured to measure deformation at a plurality of locations in the base part.

13. The apparatus of any preceding claim, wherein the deformation sensor is embedded in the force-conveying material.

14. The apparatus of any one of claims 1-13, wherein the deformation sensor is attached to a surface of the base part.

15. A method comprising
sensing deformation conveyed from a nozzle part of an apparatus comprising a body made of force-conveying material, wherein the body comprises the nozzle part and a base part, the nozzle part protruding from the base part, and wherein the nozzle part is configured to fit at least partly into a mouth of a subject for feeding the subject, and
performing the deformation sensing by at least one deformation sensor located in the base part, the deformation sensor being configured to sense, at the base part, deformation conveyed from the nozzle part through the force-conveying material.

100

102

101

103

Fig. 1A

101 110

102

103

112

113

Fig. 2

101

102 200

103

Fig. 1B

Fig. 3

Fig. 4

Fig. 5

600

102

101

301

**Fig. 6**

103

700

102

101

301

701

**Fig. 7**

103

101

800

102

820

801

103

310

Fig. 8

Start 901

Detecting deformation of the nozzle part using a deformation sensor in the base part 902

Using deformation sensed by the deformation sensor for determining forces applied to the nozzle part 903

Fig. 9

Europäisches
Patentamt

European
Patent Office

Office européen
des brevets

**EUROPEAN SEARCH REPORT**

Application Number

EP 17 16 1232

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/302924 A1 (CUNNINGHAM THOMAS J [US] ET AL) 29 November 2012 (2012-11-29) * paragraph [0045] - paragraph [0087]; figures 1A-7B * | 1-15 | INV. G01B21/32 G01B7/16 A61J13/00 |
| X | US 2015/196247 A1 (LAU CHANTAL [US]) 16 July 2015 (2015-07-16) * paragraph [0018] - paragraph [0032] * * paragraph [0077] - paragraph [0110]; figures 1-11 * * paragraph [0133] - paragraph [0138]; figures 22A-D * * paragraph [0157] - paragraph [0158] * | 1-4,7, 9-15 | ADD. G01B13/24 A61B5/03 |
| X | TAMILIA ELEONORA ET AL: "An Automated System for the Analysis of Newborns' Oral-Motor Behavior", IEEE TRANSACTIONS ON NEURAL SYSTEMS AND REHABILITATIONENGINEERING, IEEE SERVICE CENTER, NEW YORK, NY, US, vol. 24, no. 12, 12 December 2016 (2016-12-12), pages 1294-1303, XP011635959, ISSN: 1534-4320, DOI: 10.1109/TNSRE.2015.2496150 [retrieved on 2016-12-06] * II. SUCKING MONITORING DEVICE * | 1,9,11, 12,15 | |

TECHNICAL FIELDS SEARCHED (IPC)

G01B
A61J
A61B
G01L

The present search report has been drawn up for all claims

| Place of search | Date of completion of the search | Examiner |
|---|---|---|
| Munich | 26 July 2017 | Ardelt, Per-Lennart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
### ON EUROPEAN PATENT APPLICATION NO.

EP 17 16 1232

26-07-2017

| Patent document cited in search report | | Publication date | Patent family member(s) | | Publication date |
|---|---|---|---|---|---|
| US 2012302924 | A1 | 29-11-2012 | CN 103702611 A | | 02-04-2014 |
| | | | EP 2713882 A1 | | 09-04-2014 |
| | | | US 2012302924 A1 | | 29-11-2012 |
| | | | US 2014142415 A1 | | 22-05-2014 |
| | | | WO 2012166603 A1 | | 06-12-2012 |
| US 2015196247 | A1 | 16-07-2015 | EP 2874595 A1 | | 27-05-2015 |
| | | | US 2015196247 A1 | | 16-07-2015 |
| | | | US 2017188936 A1 | | 06-07-2017 |
| | | | WO 2014015180 A1 | | 23-01-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TAMILIA, E. et al.** Technological Solutions and Main Indices for the Assessment of Newborns' Nutritive Sucking: a Review. *Sensors,* 2014, vol. 14 (1), 634-658